# EUROPEAN PATENT SPECIFICATION

(11) **EP 2 102 659 B1**
(45) Date of publication and mention of the grant of the patent: **31.10.2012**
(21) Application number: 07852239.8
(22) Date of filing: 10.12.2007
(51) Int. Cl.: G01N 33/543

(54) **CONTINUOUSLY REPEATABLE METHOD OF DETECTING ANTIGENS IN TEST VOLUME**
STÄNDIG WIEDERHOLBARES VERFAHREN ZUM NACHWEIS VON ANTIGENEN IN EINEM TESTVOLUMEN
PROCEDE REPETABLE EN CONTINU DE DETECTION D'ANTIGENES DANS UN VOLUME D'ESSAI

(30) Priority: 13.12.2006 SE 0602682; 13.12.2006 US 874470 P
(43) Date of publication of application: 23.09.2009
(73) Proprietor: Biosensor Applications Sweden AB (Publ), 171 54 Solna (SE)
(72) Inventor: MÅNSSON, Per, 192 54 Sollentuna (SE); SANDÉN, Björn, 120 68 Stockholm (SE)
(74) Representative: Nikolopoulou, Sofia
(86) International application number: PCT/SE2007/050967
(87) International publication number: WO 2008/073042

(56) References cited:
- WO-A1-97/44664
- WO-A1-2004/001417
- WO-A1-2005/050209
- WO-A2-00/43774
- WO-A2-00/68419
- HALAMEK J ET AL: "Piezoelectric affinity sensors for cocaine and cholinesterase inhibitors" TALANTA, ELSEVIER, AMSTERDAM, NL, vol. 65, no. 2, 30 January 2005 (2005-01-30), pages 337-342, XP004638919 ISSN: 0039-9140

## Description

The present invention relates to a continuously repeatable method of detecting one or several different target antigens in a test volume with the aid of a flow-through analysis device with one or several flow-through testing cell(s), each containing a sensing surface, such as a Piezoelectric Quartz Crystal Microbalance device (QCM), an Ellipsometer device or a Surface Plasmon Resonance (SPR) biosensor device.

### Background

The Surface Plasmon Resonance (SPR) biosensor is a sensitive real-time technique, which can be used to extract information about molecular interaction near certain metal surfaces. It offers the possibility to determine concentration, association and dissociation rate constants and affinity as well as epitope mapping and determination of interaction specificity [B. Liedberg and K. Johansen, Affinity biosensing based on surface plasmon detection in "Methods in Biotechnology, Vol. 7: Affinity Biosensors: Techniques and Protocols", K. R. Rogers and A. Muchandani (Eds.), Humana Press Inc., Totowa, NJ, pp. 31-53]. One of the components participating in the studied reaction may be immobilized on the metal surface either before or during the SPR experiment. The immobilized molecule is exposed to a continuous flow into which one can inject interacting species. The method is based on optical detection and the sensing signal reflects changes in dielectric function or refractive index at the surface. These changes can be caused by molecular interaction at the surface.

An ellipsometer uses the change in polarization state of light that changes when reflected from a surface. When the surface composition changes in a flow cell the polarization of the reflected light is changed. Information about the film thickness may thus be deduced from such a measurement. The method can be applied in a flow cell as described in the US published patent application No. US2004/0142482.

The piezoelectric technique is based on the well-known principle of measuring the mass change in real time by measuring the frequency of a piezoelectric quartz crystal. The piezoelectric crystal device consists of a quartz crystal wafer having a metal electrode on both sides. These electrodes are used to induce an oscillating resonant frequency that is directly related to the change of the deposited mass on the electrode. Piezoelectric crystals can therefore be used for sensitive mass measurement and are therefore called Quartz Crystal Microbalances (QCM). A number of equations have been proposed to describe the relationship between frequency changes and mass deposition on the crystal.

There is a large number of patents directed to the detection of a predetermined chemical or biomolecule in a solution by use of a piezoelectric crystal microbalance, for example, US patents Nos. 4,735,906, 4,789, 804, and 5,705,399 and Halamek J et. Al. "Piezoelectric affinity sensors for cocaine and cholinesterase inhibitors" Talanta, vol. 65, no.2, 2005 p 337-342. In all known prior art using the competition reactions, the sensor surfaces are regenerated by adding an appropriate regeneration solution, e.g. acidic glycine, between the calibration analysis and the sample analysis. The sensor surfaces are also always regenerated before next calibration/analysis run. When the presence of several individual chemicals or biomolecules (analytes) are to be determined in the same test solution, then it is advantageous to have a system that can simultaneously handle a plurality of microbalances with flow cells which are individually specific for one of the analytes to be detected. Such a system has been disclosed in our International patent application WO2004001392, and it is particularly useful for screening of a large number of samples in a short time period.

The hitherto known analysis methods used for analysis of liquid samples to detect one or several target antigens therein by using specific antibodies forming complexes with the target antigen(s), have been designed for either competition reaction(s) or displacement reaction(s). The required affinity of the antibody to an immobilized antigen derivative needs to be weaker then its affinity for the target antigen in the liquid sample in displacement reactions, whereas this has not been a requirement in competition reactions.

### Description of the invention

The present invention provides a continuously repeatable method of detecting possible presence of one or several different selected target antigen(s) in a predetermined test volume with a flow-through analysis device. The analysis device is equipped with one or several flow-through testing cell(s) comprising a sensing surface. Each sensing surface is individually designed to have a modified antigen immobilized thereon which temporarily attaches an antibody that has a stronger affinity for the target antigen to be detected. Such a flow-through cell is used in the present invention in the analysis device for competition reactions. The selected target antigen in the test volume is mixed with a predetermined amount of its selective antibody.The amount of antibody that has not formed a complex with the target antigen is measured by a temporary attachment to the sensor surface that is functionalized with the modified antigen. The temporary attachment is adequate for measurement of competing antibody-antigen complexes in a test volume at the sensing surface. The antibody will after the temporary attachment dissociate from the sensing surface and will be carried away by the flowing liquid after the sample volume has passed the cell. In the prior art disclosing competition reactions, such as in our International patent application WO 2005050209, the sensing surface may be regenerated by lowering the pH of the flowing liquid e.g. by addition of glycine. When one or several of the above described flow-through cell(s) for competition reactions is (are) used in a continuous flow-through analysis device, no regeneration of the sensing surface is needed before a next test volume is analyzed according to the present invention.

The present invention also provides a continuously repeatable method detecting possible presence of one or several different selected target antigen(s) in a predetermined test volume with a flow-through analysis device wherein the device is run simultaneously with one or several individually operated flow-through cell(s) for competition reaction(s) as well as one or several individually operated flow-through cell(s) for displacement reactions.

Thus, the present invention provides a continuously repeatable method of detecting possible presence of one or several different selected target antigen(s) in a predetermined test volume comprising a buffer solution with a flow-through analysis device comprising one or several fluidly connected, individually operated, flow-through testing cells, each containing a sensing surface, each sensing surface comprising a selected modified target antigen immobilized thereon, which modified antigen has weaker affinity than the target antigen for a selected antibody specific for the selected target antigen , comprising the steps of
a) flowing the buffer solution through each testing cell,
b) registering individually a baseline value for each sensing surface,
c) flowing a predetermined volume, equal to the test volume, of the buffer solution containing a predetermined amount of one or several selected antibody or antibodies through each of the testing cell(s),
d) registering individually a weight-gain value on the sensing surface by comparison with the baseline value in b) for each sensing surface due to temporary attachment of the selected antibody to the modified target antigen in question,
e) adding to the test volume possibly containing said one or several target antigen(s) the same predetermined amount of the selected antibody or antibodies as in step c) for possible formation of target antigen-antibody complex(es),
f) flowing the test volume through each testing cell,
g) registering individually a weight value on each sensing surface, and in case there is a reduction of the weight-gain value registered in step d) it is due to less attachment of the antibody to the modified target antigen in that testing cell as a result of formation of the target antigen-antibody complex, indicating the presence of that target antigen in the test volume, followed by
h) repeating the steps a) - g) with consecutive test volumes comprising the buffer solution possibly containing said one or several target antigen(s).

For example, to detect a target antigen AgC1, such as THC or a benzodiazepine, possibly present in a test volume, by this competition mode, the flow-through analysis device is equipped with a flow-through testing cell containing a sensing surface on which a modified antigen mAgC1 is immobilized. A buffer solution is continuously flowing through the testing cell, and a baseline value is registered for the sensing surface. Then a predetermined volume, equal to the test volume, of the buffer solution containing a predetermined amount of a selected antibody AbC1, which has weaker affinity for mAgC1 than for AgC1, is run through the testing cell. A weight-gain value is registered on the sensing surface by comparison with the baseline value due to temporary attachment of AbC1 to mAgC1. Most of the attached antibodies are released from the sensing surface directly after the passage of the antibody-containing test volume due to their weak affinity to the modified antigen-containing surface. To the test volume possibly containing AgC1 is added the same predetermined amount of AbC1 for possible formation of AgC1-AbC1 complex and the test volume is run through the testing cell. The weight value on each sensing surface is registered, and in case there is a reduction of the earlier registered weight-gain value, it is due to less attachment of AbC1 to mAgC1 as a result of formation of the AgC1-AbC1 complex, indicating the presence of AbC1 in the test volume. Due to quick release of the AbC1 from the mAgC1 on the sensing surface, a next testing volume can be analyzed after a short flowing of the buffer solution, without any need for regeneration of the sensing surface. If the analysis is repeated continuously, no new baseline value is needed for a number of consecutive runs of test volumes. In this case, the steps b), c) and d) of the method of the invention are repeated only at choice or if required after the run with the first test volume, such as after the run with the second to one hundredth test volume. For example, after the run with the fifth to fiftieth test volume.

In another example, this competition mode is used for detection of an additional target antigen AgC2. Then, the flow-through analysis device is equipped with a flow-through testing cell containing a sensing surface on which a modified antigen mAgC2 is immobilized, in addition to the testing cell for detection of AgC1. The antibodies AbC1 and AbC2 are added to the same predetermined volume and the target antigens AgC1 and AgC2 are detected if they form a complex with their respective antibodies AbC1 and AbC2 due to competition in with the testing cells containing respective modified antigens mAgC1 and mAgC2.

The above two examples illustrate the detection of one target antigen and two different target antigens, respectively. However, it should be understood that the method of the invention can be used for simultaneous detection of several different target antigens, e.g. AgC3, AgC4, etc.

The method of the invention will optionally comprise an embodiment wherein the presence of one or several of the several different target antigens is (are) individually detected in the test volume by the displacement mode by adding one or several selected antibody or antibodies specific for the selected target antigen(s) to the buffer solution in step a) instead of in steps c) and e) for temporary formation of modified antigen-antibody complex(es) at the sensing surface(s) in question, and in step g) registering individually the weight value on each sensing surface, and in case there is weight-loss compared to the baseline value registered in step b), it is due to displacement of the antibody from the modified target antigen in that testing cell as a result of formation of the target antigen-antibody complex, indicating the presence of that target antigen in the test volume. In this embodiment, the flowing buffer solution is supplemented by the individual antibodies against one or several different selected target antigen(s) before it flows into each testing cell. The individual antibodies attach to the sensing surface only in cells where the modified antigen against which it has specific affinity is immobilized. When the test volume contains the antigen in question, the antigen will form a stronger complex with the antibody and displace it from the sensing surface, and the complex formed is carried away by the flowing buffer solution.

For example, by the method of the invention, in addition to detecting at least one target antigen AgC1 possibly present in a test volume by this competition mode, at least one target antigen AgD1, such as cocaine, heroin or amphetamine, is detected in the same test volume by the displacement mode. The flow-through analysis device is in this case equipped also with a flow-through testing cell containing a sensing surface on which a modified antigen mAgD1 is immobilized. A predetermined amount of a selected antibody AbD1, which has weaker affinity for mAgD1 than for AgD1, is added to the flowing buffer solution prior to the registration of a baseline value. In this case, the baseline value is taken when the AbD1 is temporarily attached to the immobilized mAgD1 on the sensing surface of the testing cell. When the test volume containing AgD1 flows through the testing cell, AgD1 displaces some of the the AbD1 from the immobilized mAgD1 as the AgD1-AbD1 complex is formed, if AgD1 is present in the test volume. This is registered as a weight loss on the sensing surface in comparison with the baseline value. The complex is carried away by the flowing buffer solution, thus leaving the sensing surface ready for a next test volume to be analyzed after a short flowing of the buffer solution. If the analysis is repeated continuously, no new baseline value is needed for a number of consecutive runs of test volumes. In this case, the addition of the antibody to the flowing buffer solution in this displacement mode is repeated at choice or if required after the run with the first test volume. In practice, it is convenient to add fresh antibodies before each new run of a test volume in this displacement mode.

In another example of this embodiment, this displacement mode is used for detection of an additional target antigen AgD2. Then, the flow-through analysis device is equipped with a flow-through testing cell containing a sensing surface on which a modified antigen mAgD2 is immobilized, in addition to the testing cell for detection of AgD1 and at least one testing cell for competition detection of AgC1. A predetermined amount of a selected antibody AbD2, which has weaker affinity for mAgD2 than for AgD2, is added to the flowing buffer solution together with the AbD1 prior to the registration of a baseline value. When the test volume containing AgD2 flows through the testing cell, AgD2 displaces the AbD2 from the immobilized mAgD2 as the AgD21-AbD21 complex is formed, if AgD2 is present in the test volume. This is registered as a weight loss on the sensing surface in comparison with the baseline value.

The above two examples illustrate the detection of two different target antigens AgD1 and AgD2, respectively, by the displacement mode at the same time as at least one target antigen AgC1 by the competition mode. However, it should be understood that the method of the invention can be used for simultaneous detection of several different target antigens, e.g. AgD3, AgD4, etc.

Thus, the method of the invention may be used for detection of one or several different antigens AgC1, AgC2, AgC3, AgC4, etc. by the competition mode in the same test volume as one or several different antigens AgD1, AgD2, AgD3, AgD4, etc. by the displacement mode.

In an embodiment of the invention, the one or several different selected target antigen(s) to be detected with the method of the invention is (are) selected from the group consisting of explosives and narcotics. For example, the explosives are selected from the group consisting of trinitrotoluene (TNT), dinitrotoluene (DNT), hexahydro-1,3,5-trinitro-1,3,5-triazine (RDX), octahydro-1,3,5,7-tetranitro-1,3,5,7-tetrazine (HMX), pentaerythritol tetranitrate (PETN), and nitroglycerine (NG), and the narcotics are selected from the group consisting of cocaine, heroin, amphetamine, methamphetamine, tetrahydrocannabinols (THC), benzodiazepines, such as chlordiazepoxide (Librium®), diazepam (Valium®), nitrazepam, Flunitrazepam (formerly marketed under the trade names Rohypnol, Clorazepate, Lorazepam,Oxazepam) and methylenedioxy-N-methylamphetamine (Ecstacy). The benzodiazepines are at least in some countries, such as Sweden, classified as narcotics.

The method of the invention is performed in a flow-through analysis device comprising one or several fluidly connected, individually operated, flow-through testing cells, each containing a sensing surface, each sensing surface comprising one or several selected modified target antigen(s) immobilized thereon, which modified antigen has weaker affinity than the target antigen for a selected antibody specific for the selected target antigen , which analysis device is selected from the group consisting of a Piezoelectric Quartz Crystal Microbalance device (QCM), an Ellipsometer device and a Surface Plasmon Resonance biosensor device (SPR).

The term "antibody" used in this specification and claims is intended to comprise any monospecific polyclonal or monoclonal antibodies, antibody fragments or derivatives thereof each with affinity for a predetermined antigen. The term antibody also comprises other affinity molecules such as various proteins and synthetic antibodies.

The antibodies can be custom made by specialized producers, bought from different suppliers or synthesized by procedures known from the literature such as from Hybridoma Technology in the Biosciences&Medicine. T.A. Springer, editor, Plenum Press, 1985.

The amount of the individual antibody or antibodies used in the method of the invention is e.g. between 0.01-0.8 mg/ml of the buffer solution, and in practice approximately 10 - 100 microliters of a buffer solution containing each antibody in a concentration of 0.005-0.1 mg/ml is used. A typical procedure for the competition analysis is to mix about 5 micrograms of the appropriate antibody or antibodies with a few nanograms of the antigen(s), e.g. THC and/or a benzodiazepine in a test volume of 100 mikroliter. After a few seconds of mixing, the test volume is introduced into the flow-through cell(s).

The buffer solution may be exemplified by a phosphate buffer (PBS) and it may additionally contain, stabilizers, solubility enhancers and/or preservatives, and can be selected based on the composed mixture of antibodies of choice by a man of ordinary skill in the art. The stabilizer can e.g. be a mixture of surfactants (e.g. Tween® 20 or Tween® 80 or similar) and/or various proteins (e.g. albumin, casein or other protective agents or blocking agents).

It should be understood that the expression "modified antigen immobilized on a sensing surface" comprises all kinds of spacer molecules between the antibody-binding antigenic site and surface, usually the metal surface, of the flow-through cell. Examples of such spacer molecules are comprised by our International patent applications W02004001417 and W020041416.

A selected modified the antigen may be obtained from the target antigen by chemical derivatization, or by enzymatic modification, e.g. by modification of the target antigen molecule by introduction of functional groups such as ester or amino groups (by removal of, or replacing the original groups) or by eliminating a part of the target antigen molecule, or introduction of new functional groups or side chains to the target antigen molecule, to reduce its affinity to the antibody.

It is of utmost importance that the affinity between the antibody and the modified antigen is optimized to give a weight loss upon a contact with a low concentration of the target antigen without too much weight loss upon contact with the buffer containing no target antigen. It is therefore very important to optimize the affinity of the antibodies to the immobilized modified antigen.

The invention enables rapid analysis of possible presence of narcotics and/or explosives with a flow-through analysis device which has one ore several fluidly connected, individually operated, testing cells that each contain a sensing surface on which one, two or more different modified antigen(s) of predetermined antigen(s) to be detected is (are) immobilized, in a screening situation e.g. at customs or airport passenger control.

Interestingly, wiping of the skin of human drug addicts with a filter, cloth or the like, and analysis performed in accordance with the present invention has given good analysis results for tested narcotics.

The method of the invention will now be illustrated by the use of a Multi-cell Piezoelectric Quartz Crystal Microbalance device (QCM) disclosed in our International patent application WO2004001392.

### Electrode preparation

The QCM-electrodes in the flow-through cells in the analysis device were prepared for displacement reaction (and used also for competition reactions) according to our co-pending International patent applications W020041416 and W020041417. Each of the gold electrodes on the piezoelectric crystals (QCM-crystals) are surface-coated with their respective modified antigens which are derivatives of predetermined target antigens that are to be detected. Each immobilized modified antigen has been chemically modified in order to show a weaker affinity to an antibody than the target antigen in buffer solution. The surface modified QCM-crystals were inserted into the cell housing (testing cell) and thereafter docked to the flowing system in the instrument. The buffer solution is pumped through the consecutive cells, which are stabilized within a few minutes.

### A typical analysis run can be described as follows:

A test volume is introduced into the testing cell(s) in the automatic instrument described in our International patent application W02004001392 by looping-in. The sample to be tested has usually been collected onto a filter by wiping a suspected surface and/or collection of surrounding vapor by using a vacuum cleaner or pre-concentrator of some kind. The target antigen(s) of the collected sample on the filter is (are) transferred and purified by means of a desorption process described in the International patent application WO 03/073070, or by means of an extraction by using an ionizer probe, such as one described in our International patent application WO2004104559, to a test volume containing a buffer solution. The different monoclonal antibodies against the different target antigens are introduced to the individual testing cells as described above prior to the test volume (the sample plug) is introduced into the flow when displacement mode is chosen for a testing cell. When competition mode is chosen for a testing cell, the antibody is added to the test volume (the sample plug).

### Results

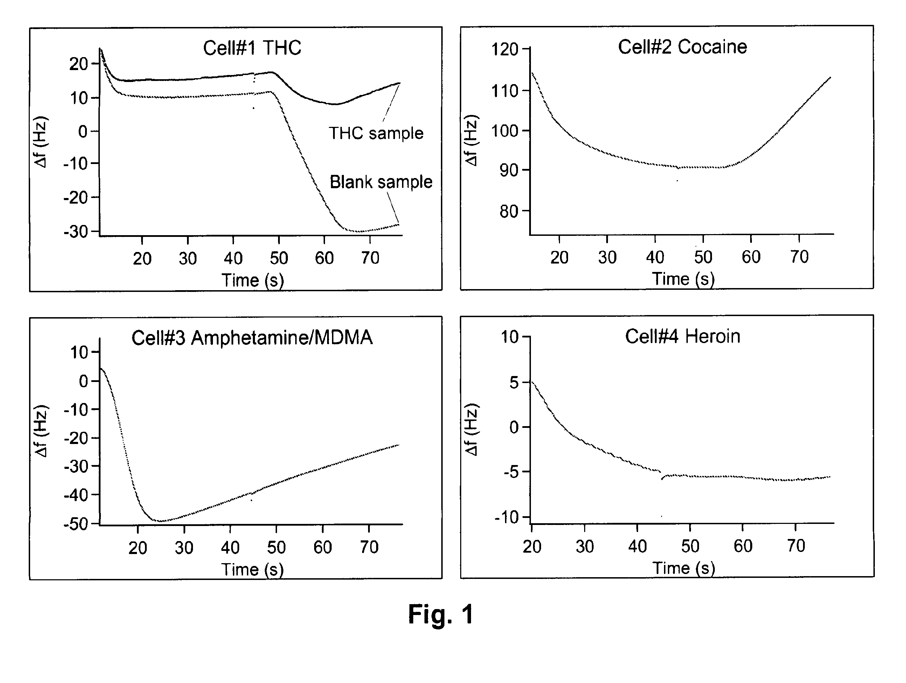

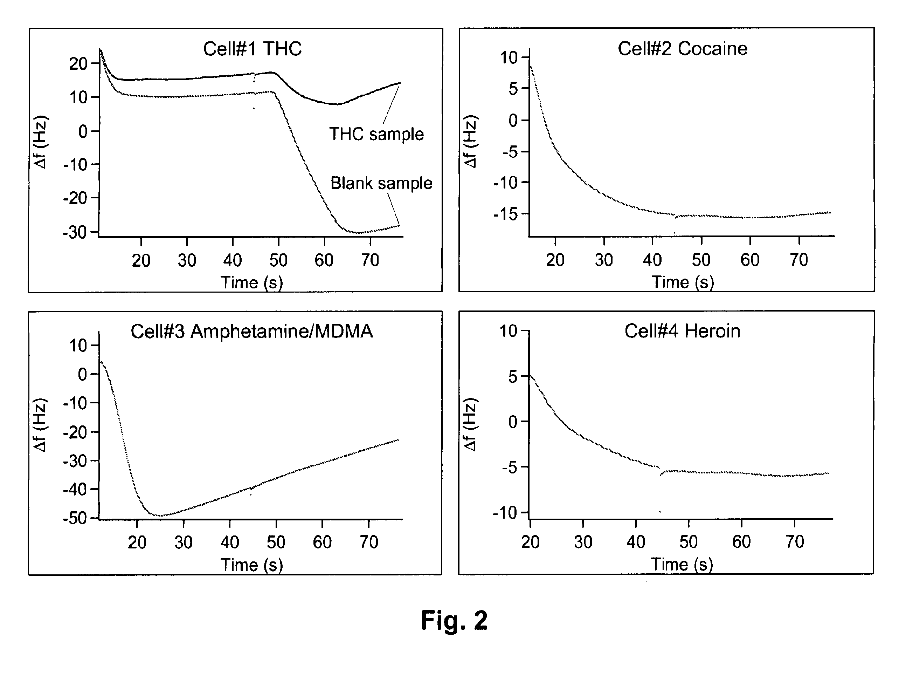

The results from a run of four fluidly serially connected testing cells for detection of THC by the competition mode, and cocaine, heroin and amphetamine, respectively, by the displacement mode are presented in figures 1 and 2.
Figure 1. Typical response curve from four different sensor surfaces (QCM-cells) that are sensitive against THC, cocaine, amphetamine and heroin. The sample plug is spiked with cocaine (20 picogram/microliter) and antibodies against THC. The antibodies against cocaine, amphetamine and heroin were added at time zero and the sample with THC-antibodies and cocaine was introduced at time 45 seconds. As can be seen in the figure, the curve from Cell#2 (cocaine) shows a pronounced weight loss. The curve in CELL#1 (THC) shows a pronounced weight gain due to the presence of THC-antibodies.
Figure 2. Typical response curve from four different sensor surfaces (QCM-cells) that are sensitive against THC, cocaine, amphetamine and heroin. The sample plug is spiked with THC (20 picograms/mikroliter) and antibodies against THC. The antibodies against cocaine, amphetamine and heroin were added at time zero and the sample with THC-antibodies and THC was introduced at time 45 seconds. As can be seen in the figure, only the THC-cell showed a pronounced weight loss of the attached antibodies. A result from a blank sample with no THC in the sample plug is shown as comparison.

## Claims

1. A continuously repeatable method of detecting
possible presence of one or several different selected target antigen(s) in a predetermined test volume comprising a buffer solution
with a flow-through analysis device comprising one or several fluidly connected, individually operated, flow-through testing cells,
each cell containing a sensing surface,
each sensing surface comprising a selected modified target antigen immobilized thereon, which modified antigen has weaker affinity than the target antigen for a selected antibody specific for the selected target antigen,
by individually designing each sensing surface in the flow-through testing cell(s) to have a modified antigen immobilized thereon which temporarily attaches an antibody that has stronger affinity for the target antigen to be detected, so that the antibody after the temporary attachment will dissociate from the sensing surface and will be carried away by the flowing liquid,
consisting of the steps of:
a) flowing buffer solution through each testing cell,
b) registering individually a baseline value for each sensing surface,
c) flowing a predetermined volume, equal to the test volume, of the buffer solution containing a predetermined amount of one or several selected antibody or antibodies through each testing cell,
d) registering individually a weight-gain value on the sensing surface by comparison with the baseline value in b) for each sensing surface due to temporary attachment of the selected antibody to the modified target antigen in question,
e) adding to the test volume possibly containing said one or several target antigen(s) the same predetermined amount of the selected antibody or antibodies as in step c) for possible formation of target antigen-antibody complex(es),
f) flowing the test volume through each testing cell,
g) registering individually a weight value on each sensing surface,
and in case there is a reduction of the weight-gain value registered in step d) it is due to less attachment of the antibody to the modified target antigen in that testing cell as a result of formation of the target antigen-antibody complex, indicating the presence of that target antigen in the test volume, followed by
h) repeating the steps a) - g) with consecutive predetermined test volumes comprising the buffer solution possibly containing said one or several target antigen(s).

2. The method according to claim 1, wherein the steps b), c) and d) are repeated only at choice, or if required, after the run with the first predetermined test volume.

3. The method according to claim 2, wherein the steps b), c) and d) are repeated after the second to one hundredth predetermined test volume.

4. The method according to any one of claims 1 - 3, wherein the analysis device is selected from the group consisting of a Piezoelectric Quartz Crystal Microbalance device (QCM), an Ellipsometer device and a Surface Plasmon Resonance biosensor device.

5. The method according to any one of claims 1 - 4, wherein the one or several different selected target antigen(s) is (are) selected from the group consisting of explosives and narcotics.

6. The method according to claim 5, wherein the explosives are selected from the group consisting of trinitrotoluene (TNT), dinitrotoluene (DNT), hexahydro-1,3,5-trinitro-1,3,5-triazine (RDX), octahydro-1 ,3,5,7-tetranitro-1 ,3,5,7-tetrazine (HMX), pentaerythritol tetranitrate (PETN), and nitroglycerine (NG).

7. The method according to claim 5, wherein the narcotics are selected from the group consisting of cocaine, heroin, amphetamine, methamphetamine, tetrahydro- cannabinols (THC), benzodiazepines and methylenedioxy-N-methylamphetamine (Ecstacy).

## Patentansprüche

1. Ständig wiederholbares Verfahren zum Nachweis von
möglichem Vorhandensein von einem oder mehreren verschiedenen ausgewählten Zielantigenen in einem vorbestimmten Testvolumen, umfassend eine Pufferlösung
mit einer Durchströmungsanalyseeinrichtung, umfassend eine oder mehrere fluidisch verbundene, einzeln betriebene, Durchströmungstestzellen,
wobei jede Zelle eine Erfassungsoberfläche enthält,
wobei jede Erfassungsoberfläche ein ausgewähltes, modifiziertes darauf imobilisiertes Zielantigen umfasst, wobei das modifizierte Antigen eine schwächere Affinität als das Zielantigen für einen ausgewählten für das ausgewählte Zielantigen spezifischen Antikörper aufweist,
indem jede Erfassungsoberfläche in der/den urchströmungstestzelle(n) einzeln gestaltet wird, damit sie ein modifiziertes darauf immobilisiertes Antigen aufweist, welches vorübergehend einen Antikörper anbindet, welcher eine stärkere Affinität für das nachzuweisende Zielantigen aufweist, so dass sich der Antikörper nach der vorübergehenden Anbindung von der Erfassungsoberfläche trennen wird und von der strömenden Flüssigkeit weggetragen wird,
bestehend aus den Schritten von:
a) einer Strömung der Pufferlösung durch jede Testzelle
b) einer Registrierung eines asislinienwertes für jede Erfassungsoberfläche einzeln betrachtet
c) einer Strömung eines vorbestimmten Volumens, gleich dem Testvolumen, der Pufferlösung, enthaltend eine vorbestimmte Menge von einem oder mehreren ausgewählten Antikörper oder Antikörpern, durch jede Testzelle,
d) einer Registrierung eines Gewichtszunahmewertes auf der Erfassungsoberfläche einzeln betrachtet durch Vergleich mit dem Basislinienwert in b) für jede Erfassungsoberfläche infolge vorübergehender Anbindung des ausgewählten Antikörpers an das betreffende modifizierte Zielantigen,
e) einer Zugabe derselben vorbestimmten Menge vom ausgewählten Antikörper oder Antikörpern wie in Schritt c) zum Testvolumen, möglicherweise enthaltend das eine oder mehrere Zielantigene, zur möglichen Bildung von einem Zielantigen-Antikörperkomplex(en),
f) einer Strömung des Testvolumens durch jede Testzelle,
g) einer Registrierung eines Gewichtswertes auf jeder Erfassungsoberftäche einzeln betrachtet,
und falls sich eine Verringerung des in Schritt d) registrierten Gewichtszunahmewertes ergibt, ist es auf weniger Anbindung des Antikörpers an das modifizierte Zielantigen in dieser Testzelle als Folge einer Bildung vom Zielantigen-Antikörperkomplex zurückzuführen, was das Vorhandensein dieses Zielantigens im Testvolumen anzeigt, gefolgt von
h) einer Wiederholung des Schritts a) - g) mit aufeinanderfolgenden vorbestimmten Testvolumen, umfassend die Pufferlösung, möglicherweise enthaltend das eine oder mehrere Zielantigene.

2. Verfahren nach Anspruch 1, wobei nach dem Ablauf des ersten vorbestimmten Testvolumens die Schritte b), c) und d) nur bei Wahl, oder falls erforderlich, wiederholt werden.

3. Verfahren nach Anspruch 2, wobei die Schritte b), c) und d) nach dem zweiten bis zum hunderten vorbestimmten Testvolumen wiederholt werden.

4. Verfahren nach irgendeinem der Ansprüche 1-3, wobei die Analyseeinrichtung aus der Gruppe ausgewählt wird, die aus einer piezoelektrischen Quarzkristall-Mikrowaage-Einrichtung (QCM), einer Ellipsometer-Einrichtung und einer Oberflächenplasmonresonanz-Biosensoreinrichtung besteht.

5. Verfahren nach irgendeinem der Ansprüche 1-4, wobei das eine oder mehrere verschiedene ausgewählte Zielantigene aus der Gruppe ausgewählt werden, die aus Explosivstoffen und Narkosemitteln besteht.

6. Verfahren nach Anspruch 5, wobei die Explosivstoffe aus der Gruppe ausgewählt werden, die aus Trinitrotoluen (TNT), Dinitrotoluen (DNT), Hexahydro-1,3,5-Trinitro-1,3,5-Triazin (RDX), Octahydro-1,3,5,7-Tetranitro-1,3,5,7-Tetrazin (HMX), Pentaerythritoltetranitrat (PETN) und Nitroglycerin (NG) besteht.

7. Verfahren nach Anspruch 5, wobei die Narkosemittel aus der Gruppe ausgewählt werden, die aus Kokain, Heroin, Amphetamin, Methamphetamin, Tetrahydro-Cannabinolen (THC), Benzodiazepinen und Methylendioxy-N-Methylamphetamin (Ecstasy) besteht.

## Revendications

1. Procédé répétable en continu de détection
de la présence éventuelle d'un ou de plusieurs différents antigènes de cible sélectionnés dans un volume d'essais prédéterminé comprenant une solution de tampon
avec un dispositif d'analyse avec renouvellement continu comprenant une ou plusieurs cellules d'essais avec renouvellement continu, reliées fluidiquement et commandées individuellement,
chaque cellule contenant une surface de détection,
chaque surface de détection comprenant un antigène de cible sélectionné, modifié et immobilisé sur celle-ci, ledit antigène modifié présentant une affinité plus faible que celle de l'antigène de cible pour un anticorps spécifique sélectionné pour l'antigène de cible sélectionné,
en concevant individuellement chaque surface de détection dans la ou les cellules d'essais avec renouvellement continu de manière à présenter un antigène modifié et immobilisé sur celle-ci qui attache temporairement un anticorps ayant une plus forte affinité pour l'antigène de cible à détecter si bien que l'anticorps après l'attachement temporaire va dissocier à partir de la surface de détection et va être emporté par le liquide courant,
comprenant les étapes consistant à:
a) l'écoulement de la solution de tampon à travers chaque cellule d'essais,
b) l'enregistrement individuel d'une valeur de référence pour chaque surface de détection,
c) l'écoulement d'un volume prédéterminé, égal au volume d'essais, de la solution de tampon contenant une quantité prédéterminée d'un ou de plusieurs anticorps choisis à travers chaque cellule d'essais,
d) l'enregistrement individuel d'une valeur de gain de poids sur la surface de détection par comparaison à la valeur de référence en b) pour chaque surface de détection en raison de l'attachement temporaire de l'anticorps sélectionné à l'antigène de cible modifié en question,
e) l'ajout au volume d'essais éventuellement contenant ledit un antigène ou lesdits plusieurs antigènes de cible de la même quantité prédéterminée de l'anticorps ou des anticorps sélectionnés qu'à l'étape c) pour la formation possible de complexe(s) d'antigène-anticorps de cible,
f) l'écoulement du volume d'essais à travers chaque cellule d'essais,
g) l'enregistrement individuel d'une valeur de poids sur chaque surface de détection,
et dans le cas où il existerait une réduction de la valeur de gain de poids enregistrée dans l'étape d), elle est due à moins d'attachement de l'anticorps à l'antigène de cible modifié en ce que la cellule d'essais en tant qu'un résultat de la formation du complexe d'antigène-anticorps de cible, indiquant la présence de cet antigène de cible dans le volume d'essais, suivi par
h) la répétition des étapes a) - g) avec des volumes d'essais consécutifs et prédéterminés comprenant la solution de tampon éventuellement contenant ledit un antigène ou lesdits plusieurs antigènes de cible.

2. Procédé selon la revendication 1, dans lequel les étapes b), c) et d) ne sont répétées qu'à choix ou si nécessaire après le parcours du premier volume d'essais prédétermine.

3. Procédé selon la revendication 2, dans lequel les étapes b), c) et d) sont répétées après le deuxième au centième volume d'essais prédéterminé.

4. Procédé selon l'une quelconque des revendications 1 à 3, dans lequel le dispositif d'analyse est choisi du groupe composé par un dispositif piézoélectrique de microbalance à cristal de quartz (Quartz Crystal Microbalance, QCM), un ellipsometer et un dispositif de biocapteur de la résonance des plasmons de surface.

5. Procédé selon l'une des revendications 1 à 4, dans lequel l'un ou plusieurs différents antigènes de cibles sont choisis du groupe composé par d'explosifs et de stupéfiants.

6. Procédé selon la revendication 5, dans lequel les explosifs sont choisis du groupe composé par le trinitrotoluène (TNT), le dinitrotoluène (DNT), l'hexahydro-1,3,5-trinitro-1,3,5- triazine (RDX), l'octahydro-1,3,5,7-tétranitro-1 ,3,5,7-tétrazocine (HMX), le tétranitrate de pentaérythritol (PETN) et la nitroglycérine (NG).

7. Procédé selon la revendication 5, dans lequel les stupéfiants sont choisis du groupe composé par la cocaïne, l'héroïne, l'amphétamine, la méthamphétamine, le tétrahydrocannabinol (THC), les benzodiazépines et méthylènedioxy-N-méthylamphétamine (Ecstacy).
